# EUROPEAN PATENT SPECIFICATION

(11) **EP 1 742 596 B1**
(45) Date of publication and mention of the grant of the patent: **02.03.2011**
(21) Application number: 04731462.0
(22) Date of filing: 06.05.2004
(51) Int. Cl.: A61F 2/06, A61M 25/10

(54) **A CATHETER FOR THE TREATMENT OF BIFURCATIONS**
KATHETER ZUR BEHANDLUNG VON GABELUNGEN
CATHETER POUR LE TRAITEMENT DE BIFURCATIONS

(43) Date of publication of application: 17.01.2007
(73) Proprietor: Invatec S.r.l, 25030 Roncadelle (Brescia) (IT)
(72) Inventor: VENTURELLI, Andrea, I-25062 Concesio (Brescia) (IT)
(74) Representative: Crippa, Paolo Ernesto
(86) International application number: PCT/IT2004/000250
(87) International publication number: WO 2005/107643

(56) References cited:
- WO-A-02/091951
- GB-A- 2 385 530
- US-A- 6 099 497
- US-B1- 6 575 994

## Description

. The present finding relates to the field of catheters for angioplasty and particularly to a catheter for the treatment of stenosis located near a bifurcation of the blood vessel, as defined in claim 1.

. The angioplasty treatment of stenosis firstly provides the use of a balloon suitable to be inserted while deflated into the small cavity left free by the plaque and then to be blown to force the plaque and restore an inner diameter of the blood vessel similar to the nominal diameter, i.e. the diameter that the same vessel would have under a non-pathological condition.

. The use of the balloons to place a stent inside the stenosis is also known. The stent is a tubular reinforcement, generally a metal web, which is fit onto the deflated balloon and is able to be plastically deformed by inflating the balloon such as to expand until the nominal diameter of the vessel. By remaining in position in its deformed configuration, the stent is thus able to support the vessel walls.

. Though these techniques have been developed to treat simple lengths of blood vessels, yet the stenosis developing near a bifurcation can already be treated in a similar manner.

. Generally, stents used in bifurcations are tubular structures made of a metal web. These tubular structures may be either plain or Y-shaped.

. Among the current systems for the treatment of bifurcations there are some providing the use of a catheter with a distal end split in two and comprising two identical balloons.

. These catheters are conceived to carry out an angioplasty treatment and to place a Y-shaped stent into the bifurcation.

. A catheter of this type suffers from some substantial drawbacks. First of all because, during the very first steps of the angioplasty operation, two deflated balloons placed side-by-side are very bulky as a whole and determine a notable irregularity of the catheter section in the distal length thereof. Due to the bulk and irregularity of its section, the catheter becomes difficult to drive through the tortuous path towards the bifurcation to be treated.

. Secondly, because during the angioplasty operation, two balloons placed side-by-side in the common vessel length, before the bifurcation, entail a damaging over-dilatation of the vessel. In fact, both balloons are sized such as to have their expanded diameter equal to the nominal diameter of the vessels to be treated.

. A solution to this drawback has been attempted by using a catheter having a distal end split in two, comprising two balloons and in which the length of the balloon intended for the side vessel is clearly shorter than the length of the balloon intended for the main vessel. The proximal end of the side balloon starts about at half the length of the main balloon, in order to prevent that the vessel may over-dilate in a prejudicial manner inside the length in common.

. This catheter is designed, such as that described above, to be able to place a Y-shaped stent in the proximal length in common and in both distal lengths at the same time.

. This type of catheter also entails a notable drawback since the overall shape of both balloons is not able to provide a correct shape of the stenosis at the bifurcation. In fact, the final shape provided to the stenosis by the catheter following the angioplasty operation, is influenced by the transition of the catheter side arm diameter at the point where the tube ends and the balloons start. This transition is placed exactly at the bifurcation point, i.e. where the need is the greatest for a regular profile of the vessel lumen in order to maintain the blood stream as undisturbed as possible.

. Examples of catheters for the angioplasty treatment of bifurcations of the type described above are also provided by the international patent applications WO 03/053507, WO 02/091951 and WO 96/34580 as well as by US patent applications US 6575994 and US 6099497.

. From what has been discussed above, the object of the present invention is to provide a catheter for the angioplasty treatment of the bifurcations which allows to overcome the drawbacks of the prior art catheters.

. This object is achieved by a catheter for angioplasty according to claim 1.

. Further details and the advantages of the invention will become apparent from the description given below with reference to the annexed drawings, being merely illustrative and non limiting, in which:

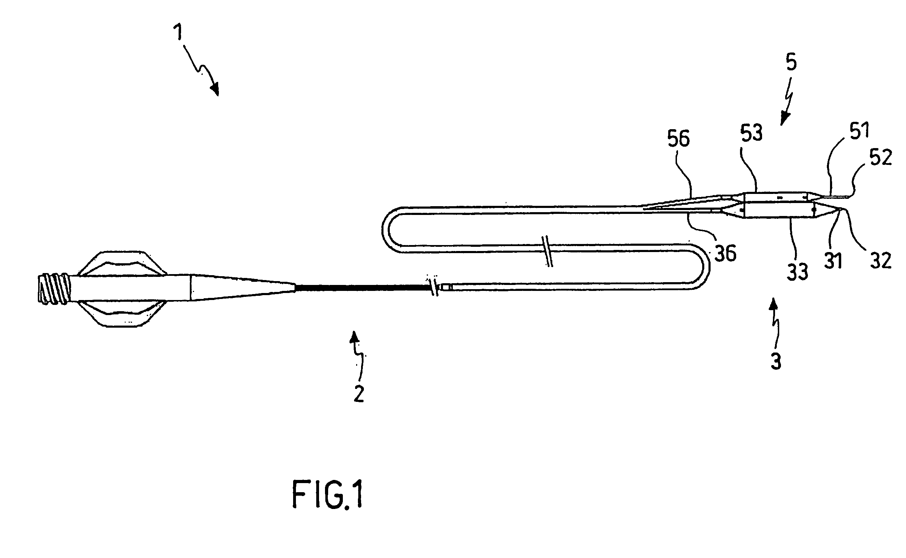

. figure 1 illustrates a side view of a catheter according to the invention;

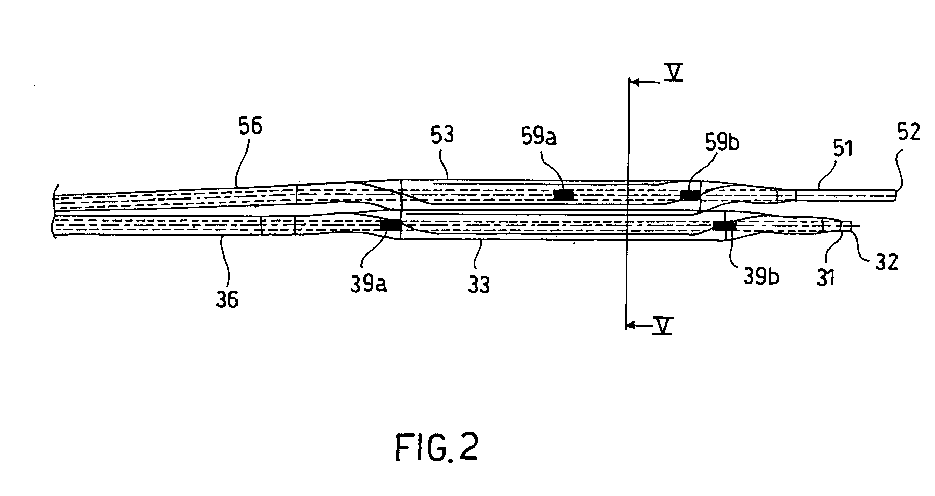

. figure 2 illustrates a side view of a detail of the distal end of the catheter from figure 1 in a first configuration;

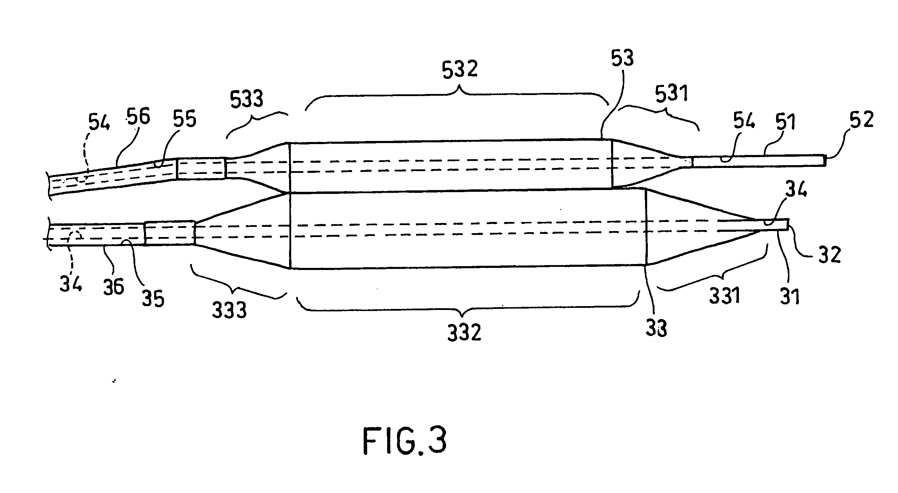

. figure 3 illustrates a side view of a detail of the distal end of the catheter from figure 1 in a second configuration;

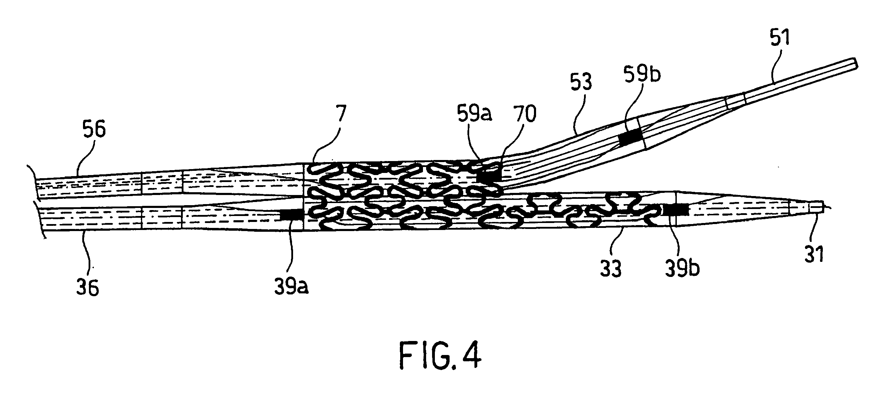

. figure 4 illustrates a side view of the detail from figure 2 with a stent loaded on the catheter;

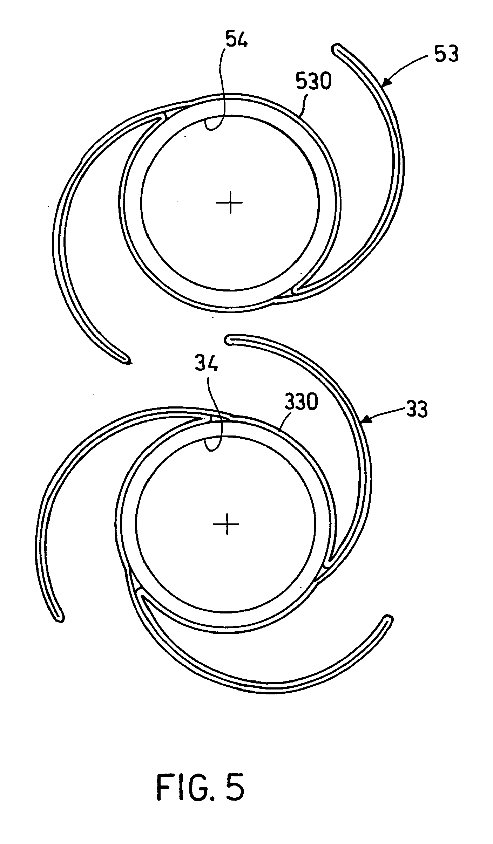

. figure 5 illustrates a section along the plan V-V from figure 2;

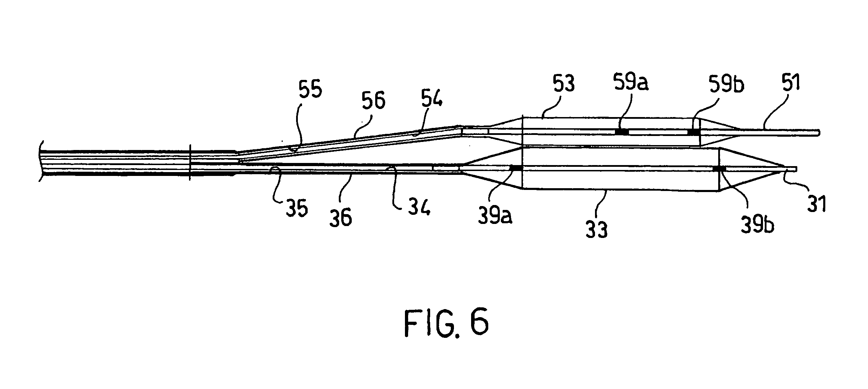

. figure 6 is a schematic illustration of a longitudinal section of a catheter according to the invention;

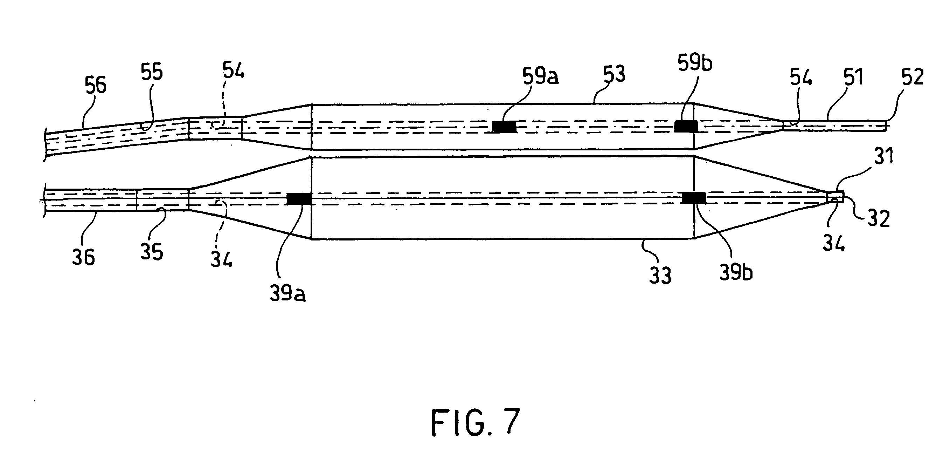

. figure 7 illustrates a detail of the distal end of another catheter according to the invention;

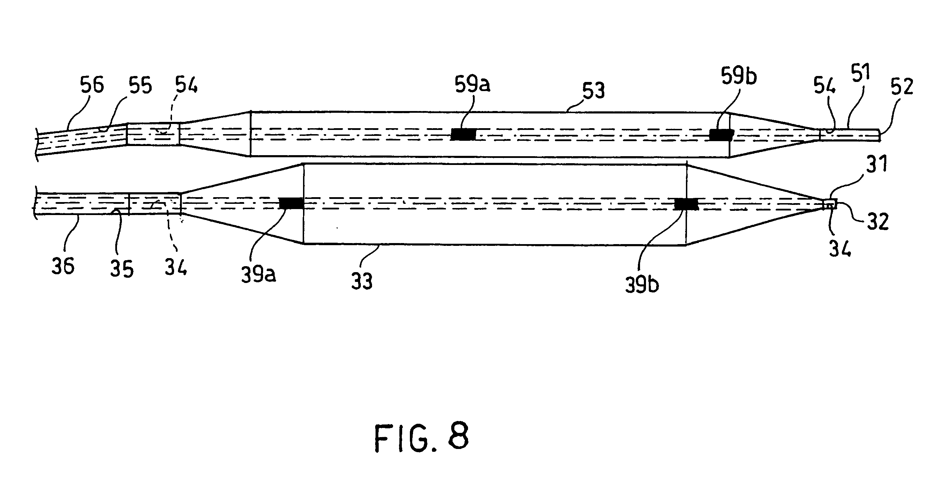

. figure 8 illustrates a detail of the distal end of a further catheter according to the invention;

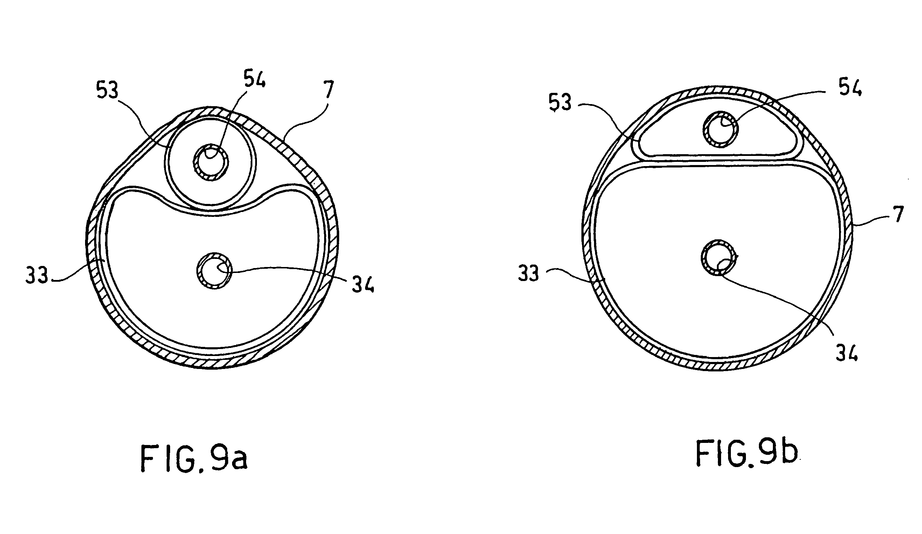

. figures 9a and 9b illustrate practical sections similar to that from figure 5 in a second configuration;

. With reference the above figures, with 1 is designated a catheter according to the invention as a whole.

. Within this catheter a proximal area 2 and a bifurcated distal area comprising a first arm 3 and a second arm 5 can be identified.

. The proximal area 2 is intended to remain external to the patient's body. It comprises the means required to connect the catheter to any complementary equipment, such as to connect the balloon inflating lumens to a source of pressurized fluid. The proximal area 2 further comprises the means required to control the movement of the catheter inside the blood vessels of the patient's body.

. The first distal arm 3 comprises in turn a first tip 31, a first balloon 33, a first tube 36, a first guide lumen 34 and a first inflating lumen 35.

. The first guide lumen 34 runs all along the tube 36, balloon 33 and tip 31 without intermediate openings. The distal end of the guide lumen 34 defines an apex port 32.

. The apex port 32 and the guide lumen 34 represent a path to a guide wire. In other words, they allow the arm 3 to slide along a guide wire suitably prearranged through the patient's blood vessels to drive the catheter from the percutaneous incision to the proper site where the angioplasty operation should be carried out.

. On the other hand, the first inflating lumen 35 is the inflating lumen of balloon 33 and the distal end thereof coincides with the proximal end of balloon 33 (see figure 6).

. The balloon 33 is shaped such that it can alternatively take either a collapsed configuration (see for example figure 2) or, following the injection of a pressurised fluid through the inflating lumen 35, an expanded configuration (see for example figure 3).

. In accordance with an embodiment, the first arm 3 comprises at least one radiopaque marker 39. In accordance with a preferred embodiment, the first arm 3 comprises two radiopaque markers 39a and 39b.

. In accordance with a preferred embodiment, the wall 330 of balloon 33 is, in its collapsed configuration, folded and rolled up on itself thus forming some flaps. In accordance with a preferred embodiment, the wall 330 forms three of such flaps, though this number may change depending on practical requirements. In this configuration, the overall bulk of the transversal section of the balloon 33 is minimized.

. On the other hand, in the expanded configuration, the wall 330 of balloon 33 deploys, the transversal section takes an almost circular profile and the diameter of the section takes its maximum value.

. Within the first balloon 33 a distal transition area 331, a middle area 332 of constant section and a proximal transition area 333 can be identified.

. The distal transition area 331 is shaped such as to join the outer diameter of the tip 31 with the outer diameter of the balloon 33.

. On the other hand, the proximal transition area 333 is shaped such as to join the outer diameter of the balloon 33 with the outer diameter of the tube 36.

. In accordance with a preferred embodiment, the distal transition area 331 and the proximal transition area 333 have the shape of a truncated cone, while the middle area 332 of constant section is cylindrical in shape.

. In accordance with a preferred embodiment, both markers 39a and 39b are located near the ends of the middle area 332 of constant section.

. The second distal arm 5 comprises in turn a second tip 51, a second balloon 53, a second tube 56, a second guide lumen 54 and a second inflating lumen 55.

. The second guide lumen 54 runs all along inside the tube 36, balloon 53 and tip 51 without intermediate openings. The distal end of guide lumen 54 defines an apex port 52.

. The apex port 52 and the guide lumen 54 represent a path to a guide wire. In other words, they allow the arm 5 to run along a guide wire suitably prearranged along the patient's blood vessels to drive the catheter from the percutaneous incision to the site where the angioplasty operation should be carried out.

. On the other hand, the second lumen 55 is an inflating lumen of balloon 53 and the distal end thereof coincides with the proximal end of the balloon 53 (see figure 6).

. The balloon 53 is shaped such as to be able to alternatively take either a collapsed configuration or, following to the injection of a pressurized fluid through lumen 55, a deployed configuration.

. In accordance to an embodiment, the second arm 5 comprises at least one radiopaque marker 59. In accordance with a preferred embodiment, the second arm 5 comprises two radiopaque markers 59a and 59b.

. In accordance with a preferred embodiment, the wall 530 of balloons 53 is, in the collapsed configuration, folded and rolled up on itself thus forming some flaps. In accordance with a preferred embodiment the wall 530 forms two of such flaps (see figure 5), yet this number may change depending on practical requirements. In this configuration, the overall bulk of the transversal section of the balloon 53 is minimized.

. On the other hand, in the expanded configuration the wall 530 of balloon 53 deploys, the transversal section takes an almost circular profile and the section diameter takes its maximum value.

. Within the second balloon 53 a distal transition section 531, a middle area 532 of constant section and a proximal transition area 533 can be identified.

. The distal transition area 531 is shaped such as to join the outer diameter of the tip 51 with the outer diameter of the balloon 53.

. On the other hand, the proximal transition area 533 is shaped such as to join the outer diameter of the balloon 53 with the outer diameter of the tube 56.

. In accordance with a preferred embodiment, the distal transition area 531 and the proximal transition area 533 have the shape of a truncated cone, while the middle area 532 of constant section is cylindrical in shape.

. In accordance with a preferred embodiment, the markers 59a and 59b are located in the centre of the middle area 532 of constant section and near the distal end thereof, respectively.

. The different arrangement of markers 39a and 39b of first arm 3 and of markers 59a and 59b of the second arm 5, enables the operators to identify both arms while performing the angiography vision.

. The second balloon 53, when being in the expanded configuration, is of a smaller diameter than the first balloon 33, also being in the expanded configuration.

. In accordance with an embodiment, the expanded diameter of the second balloon 53 is either equal to or lower than half the expanded diameter of the first balloon 33.

. In accordance with a preferred embodiment, the difference between the diameters of both balloons is such that, while being in the expanded configuration and when both balloons are encircled by an outer tubular structure such as a vessel and/or a stent, the profile of the second balloon 53 is substantially incorporated into the profile of the first balloon 33 (see figures 9a and 9b).

. In accordance with another preferred embodiment of the catheter 1, the inflating lumens 35 and 55 are made such as to allow both balloons to be inflated with different pressures. Thanks to this solution the effect of incorporating the profile of the second balloon 53 in the profile of the first balloon 33 can be enhanced.

. From what has been discussed above it should be observed how the overall profile taken by the length where both balloons are placed side-by-side, is either rounded or substantially circular.

. Due to the smaller expanded diameter of balloon 53 its bulk is reduced also when being in the collapsed configuration. In fact the flaps obtainable by folding the wall 530 on itself have peripheral widths such as not overlap one another (see figure 5). Thus, the collapsed diameter of the second balloon 53 is either substantially equal to or smaller than the part of tube 56 which is immediately adjacent thereto in the proximal direction.

. Thanks to the small bulk of the second arm 5 when the balloon 53 is collapsed, the overall handiness of catheter 1 during the manoeuvres preliminary to the operation of angioplasty is clearly better than a known catheter with two conventional balloons of the same size.

. Moreover, due to the small size taken by the second balloon 53 in its expanded configuration, the damaging over-dilation is avoided in the common bifurcation length, where both balloons 33 and 53 are placed side-by-side and deployed at the same time.

. In accordance with an embodiment (illustrated in figures 1 to 3) the proximal transition area 333 of first balloon 33 and the proximal transition area 533 of the second balloon 53 are axially arranged along their respective arms such that the proximal ends of the corresponding areas of constant section, 332 and 532 respectively, coincide.

. The area of constant section 532 of the second balloon 53, distally ends before the end of the area of constant section 332 of the first balloon 33.

. In accordance with another embodiment (illustrated in figure 7), similarly to what has been discussed above, the proximal transition areas 333 and 533 are arranged such that the proximal ends of the corresponding areas of constant section, 332 and 532 respectively, coincide. On the other hand, contrarily to what has been described above, in the embodiment illustrated in figure 7 the length of the areas of constant section 332 and 532 is the same, hence their distal ends also coincide.

. In accordance with a further embodiment (represented in figure 8) the proximal transition areas 333 and 533 are arranged such as to cause their proximal ends to coincide. Accordingly, the area of constant section 532 of the second balloon 53 starts at a point preceding, in the proximal direction, the point where the area of constant section 332 of the first balloon 33 starts.

. In the distal direction, two balloons 33 and 53 can be shaped such as to either cause the distal ends of the areas of constant section 332 and 532 to coincide or, such as in figure 8, such as to cause the distal ends of the transition areas 331 and 531 to coincide.

. Due to the fact that both balloons 33 and 53 have their proximal ends substantially coincident, notable advantages can be obtained in terms of overall shape taken by both balloons at the bifurcation. In fact, the final shape provided by the catheter to the stenosis following the operation of angioplasty, is not influenced - at the bifurcation - by the transition of the diameter of arm 5 at the point where the tube 56 ends and the balloon 53 starts.

. By placing this transition upstream of the bifurcation a regular profile of the vessel lumen can be ensured in order to keep the blood stream as undisturbed as possible. The bifurcation is, indeed, the point where the need is the greatest for a regular profile of the lumen.

. Regardless of the various balloon lengths as described above, the second arm 5 can be made longer than the first arm 3. In fact, the tip 52 of second arm 5 can be caused to extend beyond the apex port 31 of first arm 3.

. This peculiar structural characteristic, i.e. that of prearranging the second arm 5 tip longer than that of first arm 3, allows to obtain notable advantages while performing the operation.

. In fact, the second arm 5 with the lower diameter balloon is intended to engage the side branch of the bifurcation. Being longer, the tip 52 of second arm 5 is the first to reach the bifurcation and engages the side branch before the tip 32 of first arm 3 reaches the bifurcation. Thus, the catheter distal section spontaneously takes the proper angular bias within the main vessel, with no risk of reaching the bifurcation with an erroneous angular bias which would force the operator to repeat the positioning.

. Furthermore, due to the second tip 51 being longer, the force peak required by the operator to force the catheter inside the stenosis is focused on the second tip 51, thus favouring the insertion of the same. Only later on, when the second tip 51 is already inserted into the stenosis of the second arm, a further force peak is required to force also the first tip 31 inside the stenosis of the bifurcation first arm. Thus, both force peaks are separated and never coincide, thereby limiting the absolute value of the maximum force required.

. The catheter 1 can be a part of a kit also comprising a stent 7, mounted on the distal section of the catheter itself (see figure 4). This stent 7 is suitable to be placed inside the vessel length in common and the first distal length of the bifurcation, in order to support the walls of the vessel itself after the operation of angioplasty.

. In accordance with a preferred embodiment of the kit, the stent 7 is a plain tubular structure the walls of which are made of a metal web. In other words, the stent 7 is not Y-shaped as is common to those stents used in the treatment of bifurcations.

. The proximal length of the stent 7, intended to be placed inside the vessel length in common upstream of the bifurcation, is fit on both balloons 33 and 53. At the end of this length, the second arm 5 is caused to exit on one side from a mesh 70 of the stent 7. Thus, the stent distal length, intended to be placed in the first distal arm downstream of the bifurcation, is fitted only on the first balloon 33 (see figure 4).

. This type of solution is particularly effective because, due to the fact of being small sized, the second balloon 53 is not suitable either to accomplish a proper operation of angioplasty or to place a stent in the second distal arm of the bifurcation.

. In other words, due to its small expanded diameter, the second balloon 53 is not able to deploy to the extent of forcing the plaque and restore an inner diameter similar to the nominal diameter inside the second arm of the bifurcation.

. Again, due to its small expanded diameter, the second balloon 53 is not able to place a stent inside the second distal length of the bifurcation. In fact, the inflation of the second balloon 53 is not sufficient to plastically deform the stent to the extent of deploying it up to the nominal diameter of the vessel.

. This solution is particularly effective as it allows an optimum treatment of the proximal length in common, the first distal length and above all the bifurcation itself.

. As to the second distal length of the bifurcation, using the catheter according to the invention, an easy access of a second catheter intended only for the treatment of the second distal tract is ensured. The presence and the inflation of the second balloon 53, in fact, ensure that a cavity in the plaque will be maintained and/or formed, which will be sufficiently wide to allow the easy access of a further catheter.

. An advantage of the present invention is to provide a catheter which is able to treat the stenosis without over-dilate the length in common in order to prevent any damage to the vessel walls.

. Another advantage of the present invention is that it helps to provide the restored section of the vessel with a profile as even as possible at the bifurcation, in order to leave the blood stream undisturbed.

. A further advantage of the present invention is to have an even section with a small bulk. Indeed, the catheter according to the invention can be easily driven along the tortuous path, which in the first steps of the operation is to be lead to the bifurcation to be treated.

. To the preferred embodiments of the catheter and the kit described above, those skilled in the art, aiming at satisfying contingent and specific needs, may carry out a number of modifications, variants and replacements of elements with others functionally equivalent, without departing from the scope of the claims below

## Claims

1. Catheter (1) for the angioplasty treatment of a bifurcation;
said bifurcation comprising:
• a proximal length in common,
• a first distal length,
• a second distal length; and
• a stenosis located on the inner walls of said bifurcation;
said catheter (1) comprising:
• a proximal end (2);
• a first distal end (3) comprising a first apex tip (31) and a first balloon (33) suitable to alternatively take either an expanded configuration or a collapsed configuration, said first balloon (33):
- having a first length suitable to extend along said proximal length in common and along said first distal length of said bifurcation at the same time and
• a second distal end (5) comprising a second apex tip (51) and a second balloon (53) suitable to alternatively take either an expanded configuration or a collapsed configuration, said second balloon:
- having a second length suitable to extend along said proximal length in common and along said second distal length of said bifurcation ;
**wherein** the first **distal end** (3) comprises two radiopaque markers (39a, 39b),
**characterized in that**
the second **distal end** (5) comprises two radiopaque markers (59a, 59b)**, and**
the distance between the two radiopaque markers (39a, 39b) of the first **distal end** (3) is greater than the distance between the two radiopaque markers (59a, 59b) of the second **distal end** (5).

2. Catheter (1) according to the preceding claim , wherein the first balloon (33) comprises a distal transition area (331), a middle area (332) of constant section and a proximal transition area (333), and wherein the two radiopaque markers (39a, 39b) are located at the ends of said middle area of constant section (332).

3. Catheter (1) according to claim 2
wherein the second balloon (53) comprises a distal transition area (531), a middle area (532) of constant section and a proximal transition area (533), and wherein the two radiopaque markers (59a, 59b) are located halfway and at the distal end of said middle area of constant section (532), respectively.

4. Catheter (1) in accordance with any of the preceding claims wherein said first distal end (3) comprises a first guide lumen (34)**, and**
said second distal end (5) comprises a second guide lumen (54)**, and**
said first distal end (3) comprises a first inflating lumen (35)**, and**
said second distal end (5) comprises a second inflating lumen (55)**, and**
said first distal end (3) comprises a first apex port (32)**, and**
said second distal end (5) comprises a second apex port (52).

5. Catheter (1) in accordance with claim 3
wherein the proximal ends of said proximal transition areas (333, 533) coincide.

6. Catheter (1) in accordance with claim 3
wherein the proximal ends of said middle areas of constant section (332, 532) coincide.

7. Catheter (1) in accordance with claim 3
wherein the distal ends of said middle areas of constant section (332, 532) coincide.

8. Catheter (1) in accordance with claim 3
wherein the distal ends of said distal transition areas (331, 531) coincide.

9. Catheter (1) in accordance with claim 3 wherein said proximal transition areas (333, 533) have the shape of a truncated cone**, and**
said distal transition areas (331, 531) have the shape of a truncated cone**, and**
said middle areas of constant section (332, 532) have a cylindrical shape.

10. Catheter (1) in accordance with claim 3 wherein the diameter of said second middle area of constant section (532) in the expanded configuration is smaller than the diameter of said first middle area of constant section (332) in the expanded configuration.

11. Catheter (1) in accordance with claim 3 wherein the diameter of said second middle area of constant section (532) in the expanded configuration is equal to half the diameter of said first middle area of constant section (332) in the expanded configuration.

12. Catheter (1) in accordance with claim 3 wherein the diameter of said second middle area of constant section (532) in its expanded configuration is smaller than half the diameter of said first middle area of constant section (332) in the expanded configuration.

13. Catheter (1) in accordance with any of the preceding claims wherein said second distal end (5) is longer than said first distal end (3).

14. Catheter (1) in accordance with any of the preceding claims wherein said second apex tip (51) is longer than said first apex tip (31).

15. Catheter (1) in accordance with claim 4 wherein said inflating lumens (35, 55) are made such as to allow for said two balloons to be inflated with different pressures.

16. Kit comprising a catheter (1) in accordance with any of the preceding claims and a stent (7).

17. Kit comprising a catheter (1) in accordance with any of claims 1 to **15** and a stent (7) wherein said stent (7) is fitted along a proximal length thereof on both said balloons (33, 53).

18. Kit comprising a catheter (1) in accordance with any of claims 1 to **15** and a stent (7) wherein said stent (7) is fitted along a distal length thereof only on said first balloon (33).

## Patentansprüche

1. Katheter (1) für die Angioplastie einer Gefäßverzweigung;
wobei die Gefäßverzweigung enthält:
- eine gemeinsame proximale Länge,
- eine erste distale Länge,
- eine zweite distale Länge und
- eine Stenose, die sich an den Innenwänden der Gefäßverzweigung befindet,
wobei der Katheter (1) enthält:
- ein proximales Ende (2)
- ein erstes distales Ende (3), das eine erste Scheitelspitze (31) und einen ersten Ballon (33) enthält, der sich dazu eignet, alternativ entweder eine ausgedehnte Form oder eine kollabierte Form anzunehmen, wobei der erste Ballon (33) verfügt über:
- eine erste Länge, die sich dazu eignet, sich gleichzeitig entlang der gemeinsamen proximalen Länge und entlang der ersten distalen Länge der Gefäßverzweigung zu erstrecken, und
- ein zweites distales Ende (5), das eine zweite Scheitelspitze (51) und einen zweiten Ballon (53) enthält, der sich dazu eignet, alternativ entweder eine ausgedehnte Form oder eine kollabierte Form anzunehmen, wobei der zweite Ballon verfügt über:
- eine zweite Länge, die sich dazu eignet, sich entlang der gemeinsamen proximalen Länge und entlang der zweiten distalen Länge der Gefäßverzweigung zu erstrecken,
wobei das erste distale Ende (3) zwei röntgendichte Markierungen (39a, 39b) enthält.
**dadurch gekennzeichnet, dass**
das zweite distale Ende (5) zwei röntgendichte Markierungen (59a, 59b) enthält und
der Abstand zwischen den beiden röntgendichten Markierungen (39a, 39b) des ersten distalen Endes (3) größer ist als der Abstand zwischen den beiden röntgendichten Markierungen (59a, 59b) des zweiten distalen Endes (5).

2. Katheter (1) gemäß dem vorhergehenden Anspruch, bei dem der erste Ballon (33) einen distalen Übergangsbereich (331), einen Mittelbereich (332) eines gleichbleibenden Querschnittes und einen proximalen Übergangsbereich (333) enthält und sich die beiden röntgendichten Markierungen (39a, 39b) an den Enden des Mittelbereiches eines gleichbleibenden Querschnittes (332) befinden.

3. Katheter (1) nach Anspruch 2, bei dem der zweite Ballon (53) einen distalen Übergangsbereich (531), einen Mittelbereich (532) eines gleichbleibenden Querschnittes und einen proximalen Übergangsbereich (533) enthält und die beiden röntgendichten Markierungen (59a, 59b) in der Mitte bzw. an dem distalen Ende des Mittelbereiches eines gleichbleibenden Querschnittes (532) angeordnet sind.

4. Katheter (1) nach einem der vorhergehenden Ansprüche, bei dem das erste distale Ende (3) ein erstes Führungslumen (34) enthält und das zweite distale Ende (5) ein zweites Führungslumen (54) enthält,
das erste distale Ende (3) ein erstes Aufblaslumen (35) enthält,
das zweite distale Ende (5) ein zweites Aufblaslumen (55) enthält,
das erste distale Ende (3) einen ersten Scheitelanschluss (32) enthält und
das zweite distale Ende (5) einen zweiten Scheitelanschluss (52) enthält.

5. Katheter (1) nach Anspruch 3, bei dem die proximalen Enden der proximalen Übergangsbereiche (333, 533) übereinstimmen.

6. Katheter (1) nach Anspruch 3, bei dem die proximalen Enden der Mittelbereiche eines gleichbleibenden Querschnittes (332, 532) übereinstimmen.

7. Katheter (1) nach Anspruch 3, bei dem die distalen Enden der Mittelbereiche eines gleichbleibenden Querschnittes (332, 532) übereinstimmen.

8. Katheter (1) nach Anspruch 3, bei dem die distalen Enden des distalen Übergangsbereiches (331, 531) übereinstimmen.

9. Katheter (1) nach Anspruch 3, bei dem die proximalen Übergangsbereiche (333, 533) die Form eines Kegelstumpfes haben,
die distalen Übergangsbereiche (331, 531) die Form eines Kegelstumpfes haben und
die Mittelbereiche eines gleichbleibenden Querschnittes (332, 532) eine zylindrische Form haben.

10. Katheter (1) nach Anspruch 3, bei dem der Durchmesser des zweiten Mittelbereiches eines gleichbleibenden Querschnittes (532) im ausgedehnten Zustand kleiner ist als der Durchmesser des ersten Mittelbereiches eines gleichbleibenden Querschnittes (332) im ausgedehnten Zustand.

11. Katheter (1) nach Anspruch 3, bei dem der Durchmesser des zweiten Mittelbereiches eines gleichbleibenden Querschnittes (532) im ausgedehnten Zustand gleich der Hälfte des Durchmessers des ersten Mittelbereiches eines gleichbleibenden Querschnittes (332) im ausgedehnten Zustand ist.

12. Katheter (1) nach Anspruch 3, bei dem der Durchmesser des zweiten Mittelbereiches eines gleichbleibenden Querschnittes (532) in seinem ausgedehnten Zustand kleiner als die Hälfte des Durchmessers des ersten Mittelbereiches eines gleichbleibenden Querschnittes (332) im ausgedehnten Zustand ist.

13. Katheter (1) nach einem der vorhergehenden Ansprüche, bei dem das zweite distale Ende (5) länger ist als das erste distale Ende (3).

14. Katheter (1) nach einem der vorhergehenden Ansprüche, bei dem die zweite Scheitelspitze (51) länger als die erste Scheitelspitze (31) ist.

15. Katheter (1) nach Anspruch 4, bei dem die Aufblaslumen (35, 55) derart beschaffen sind, dass sie es gestatten, die beiden Ballone mit unterschiedlichen Drücken aufzublasen.

16. Einrichtung, enthaltend einen Katheter (1) nach einem der vorhergehenden Ansprüche und einen Stent (7).

17. Einrichtung, enthaltend einen Katheter (1) nach einem der Ansprüche 1 bis 15 und einen Stent (7), wobei der Stent (7) entlang einer proximalen Länge desselben an beiden Ballonen (33, 53) eingefügt ist.

18. Einrichtung, enthaltend einen Katheter (1) nach einem der Ansprüche 1 bis 15 und einen Stent (7), wobei der Stent (7) entlang einer distalen Länge desselben nur an dem ersten Ballon (33) eingefügt ist.

## Revendications

1. Cathéter (1) pour le traitement d'angioplastie d'une bifurcation ;
ladite bifurcation comprenant :
une partie proximale en commun,
une première partie distale,
une seconde partie distale ; et
une sténose située sur les parois intérieures de ladite bifurcation ;
ledit cathéter (1) comprenant :
une extrémité proximale (2) ;
une première extrémité distale (3) comprenant une première pointe (31) et un premier ballonnet (33) convenant pour prendre alternativement une configuration gonflée ou une configuration recroquevillée, ledit premier ballonnet (33) :
- ayant une première partie convenant pour s'étendre le long de ladite partie proximale en commun et le long de ladite première partie distale de ladite bifurcation en même temps et
une seconde extrémité distale (5) comprenant une seconde pointe (51) et un second ballonnet (53) convenant pour prendre alternativement soit une configuration gonflée ou une configuration recroquevillée, ledit second ballonnet :
- ayant une seconde partie convenant pour s'étendre le long de ladite partie proximale en commun et le long de ladite seconde partie distale de ladite bifurcation ;
dans lequel la première extrémité distale (3) comprend deux marqueurs radio-opaques (39a, 39b),
**caractérisé en ce que** la seconde extrémité distale (5) comprend deux marqueurs radio-opaques (59a, 59b), et
la distance entre les deux marqueurs radio-opaques (39a, 39b) de la première extrémité distale (3) est supérieure à la distance entre les deux marqueurs radio-opaques (59a, 59b) de la seconde extrémité distale (5).

2. Cathéter (1) selon la revendication précédente, dans lequel le premier ballonnet (33) comprend un surface de transition distale (331), une surface médiane (332) de section constante et une surface de transition proximale (333), et dans lequel les deux marqueurs radio-opaques (39a, 39b) sont situés aux extrémités de ladite surface médiane (332) de section constante.

3. Cathéter (1) selon la revendication 2 dans lequel le second ballonnet (53) comprend une surface de transition distale (531), une surface médiane (532) de section constante et une surface de transition proximale (533), et dans lequel les deux marqueurs radio-opaques (59a, 59b) sont situés respectivement à mi-chemin et à l'extrémité distale de ladite surface médiane (532) de section constante.

4. Cathéter (1) selon l'une quelconque des revendications précédentes dans lequel ladite première extrémité distale (3) comprend une première lumière de guidage (34), et
ladite seconde extrémité distale (5) comprend une seconde lumière de guidage (54), et
ladite première extrémité distale (3) comprend une première lumière de gonflage (35), et
ladite seconde extrémité distale (5) comprend une seconde lumière de gonflage (55), et
ladite première extrémité distale (3) comprend un premier orifice de pointe (32), et
ladite seconde extrémité distale (5) comprend un second orifice de pointe (52).

5. Cathéter (1) selon la revendication 3 dans lequel les extrémités proximales desdites surfaces de transition proximales (333, 533) coïncident.

6. Cathéter (1) selon la revendication 3 dans lequel les extrémités proximales desdites surfaces médianes de section constante (332, 532) coïncident.

7. Cathéter (1) selon la revendication 3 dans lequel les extrémités distales desdites surfaces médianes de section constante (332, 532) coïncident.

8. Cathéter (1) selon la revendication 3 dans lequel les extrémités distales desdites surfaces de transition distales (331, 531) coïncident.

9. Cathéter (1) selon la revendication 3 dans lequel lesdites surfaces de transition proximales (333, 533) ont la forme d'un cône tronqué, et
lesdites surfaces de transition distales (331, 531) ont la forme d'un cône tronqué, et
lesdites surfaces médianes de section constante (332, 532) ont une forme cylindrique.

10. Cathéter (1) selon la revendication 3 dans lequel le diamètre de ladite seconde surface médiane de section constante (532) dans la configuration gonflée est plus petit que le diamètre de ladite première surface médiane de section constante (332) dans la configuration gonflée.

11. Cathéter (1) selon la revendication 3 dans lequel le diamètre de ladite seconde surface médiane de section constante (532) dans la configuration gonflée est égal à la moitié du diamètre de ladite première surface médiane de section constante (332) dans la configuration gonflée.

12. Cathéter (1) selon la revendication 3 dans lequel le diamètre de ladite surface médiane de section constante (532) dans sa configuration gonflée est plus petit que la moitié du diamètre de ladite première surface médiane de section constante (332) dans la configuration gonflée.

13. Cathéter (1) selon l'une quelconque des revendications précédentes dans lequel ladite seconde extrémité distale (5) est plus longue que ladite première extrémité distale (3).

14. Cathéter (1) selon l'une quelconque des revendications précédentes dans lequel ladite seconde pointe (51) est plus longue que ladite première pointe (31).

15. Cathéter (1) selon la revendication 4 dans lequel lesdites lumières de gonflage (35, 55) sont faites de façon à permettre auxdits deux ballonnets d'être gonflés avec des pressions différentes.

16. Kit comprenant un cathéter (1) selon l'une quelconque des revendications précédentes est un stent (7).

17. Kit comprenant un cathéter (1) selon l'une quelconque des revendications 1 à 15 et un stent (7) dans lequel ledit stent (7) est monté le long d'une partie proximale de celui-ci sur les deux ballonnets (33, 53).

18. Kit comprenant un cathéter (1) selon l'une quelconque des revendications 1 à 15 et un stent (7) dans lequel ledit stent (7) est monté le long d'une partie distale de celui-ci seulement sur ledit premier ballonnet (33).
